# FASCICULE DE BREVET EUROPEEN

(11) **EP 1 091 746 B1**
(45) Date de publication et mention de la délivrance du brevet: **30.01.2002**
(21) Numéro de dépôt: 99926567.1
(22) Date de dépôt: 29.06.1999
(51) Int. Cl.: A61K 35/08

(54) **UTILISATION DE SOLUTIONS SALINES ISOOSMOTIQUES POUR LA PREPARATION DE MEDICAMENTS CERUMENOLYTIQUES**
VERWENDUNG VON ISOOSMOTISCHE SALZLÖSUNGEN ZUR HERSTELLUNG VON CERUMENOLYTISCHER PHARMAZEUTISCHER PRÄPARATE
USE OF ISO-OSMOTIC SALINE SOLUTIONS FOR PREPARING CERUMENOLYTIC MEDICINES

(30) Priorité: 29.06.1998 FR 9808249
(43) Date de publication de la demande: 18.04.2001
(73) Titulaire: LABORATOIRES GOEMAR S.A., 35400 Saint-Malo (FR)
(72) Inventeur: YVIN, Jean-Claude, F-35400 Saint Malo (FR); CADUDAL, Isabelle, F-35540 Plerguer (FR); TABARY, Olivier, F-51100 Reims (FR)
(74) Mandataire: Koch, Gustave
(86) Numéro de dépôt international: FR9901564
(87) Numéro de publication internationale: WO0000208

(56) Documents cités:
- EP-A- 0 747 044
- FR-A- 2 688 133
- FR-A- 2 735 980

## Description

L'invention a pour objet un médicament céruménolytique, c'est-à-dire un médicament propre à dissoudre les bouchons de cérumen.

On rappelle que le cérumen est une substance jaune-brun, très grasse qui, en s'agglomérant, forme des bouchons et qui est secrétée par les glandes sébacées dont est tapissé le conduit auditif externe qui relie le pavillon de l'oreille au tympan.

Les bouchons de cérumen causent des bourdonnements d'oreilles et perturbent l'audition.

On connaît déjà des médicaments et des préparations céruménolytiques, c'est-à-dire des produits destinés à la dissolution des bouchons de cérumen existants et à la prévention de la formation de nouveaux bouchons.

On connaît plus particulièrement une préparation céruménolytique à base d'eau de mer purifiée et stérilisée d'au moins 36 g de sel par litre, commercialisée sous la marque AUDISPRAY par la société DIEPHA.

Les médicaments et préparations céruménolytiques existants donnent généralement satisfaction, mais leur efficacité est perfectible.

Et la Société Demanderesse a le mérite d'avoir mis au point, à l'issue de travaux de recherche approfondis, un médicament céruménolytique d'une efficacité nettement supérieure à celle des produits céruménolytiques qui existent déjà.

Le médicament conforme à l'invention est caractérisé par le fait qu'il est à base d'une solution saline isoosmotique.

Cette solution saline isoosmotique est caractérisée :
- par un pH de 7,8 à 8,3
- par une densité de 1,008 et 1,01
- par une teneur en matières sèches de 1 à 2% en poids et
- par une constitution chimique résultant, pour ce qui est de ses principaux éléments, du tableau A ci-après :

**TABLEAU A**

| | |
|---|---|
| Solution (Na) | de 2000 à 2600 mg/l |
| Potassium (K) | de 40 à 80 mg/l |
| Chlorures (Cl) | de 5800 à 600 mg/l |
| Calcium (Ca) | de 300 à 400 mg/l |
| Magnésium (Mg) | de 1200 à 1500 mg/l |

En conséquence, l'invention réside dans l'utilisation d'une solution saline isoosmotique, notamment obtenue à partir d'eau de mer, pour l'obtention d'un médicament destiné à un traitement céruménolytique.

Pour préparer cette solution isoosmotique, on peut procéder de la manière indiquée ci-après.

On utilise comme matière première une eau de mer prélevée avantageusement par une profondeur de 5 à 10 mètres dans une zone à forts mouvements de courant, et caractérisée par une teneur en sels supérieure à 32 g/l.

Cette eau est analysée, décantée puis rapidement:
- désodée par la technique d'électrodialyse jusqu'à ce que l'isotonicité soit d'environ 9 équivalents-grammes de chlorure de sodium au litre,
- filtrée et
- stockée dans des conditions stériles en cuve en acier inoxydable.

Elle est de nouveau analysée à ce stade pour vérifier :
- sa stérilité,
- son isotonicité (physiologique).

Enfin, elle est conditionnée de façon stérile dans un local spécialement traité en atmosphère contrôlée.

Les travaux qui ont permis de mettre en évidence les propriétés céruménolytiques des solutions salines isoosmotiques et leur supériorité vis-à-vis des médicaments et préparations céruménolytiques qui existent déjà vont être décrits ci-après.

Dans le cadre de ces travaux, on a examiné l'effet céruménolytique sur des bouchons de cérumen humains en faisant agir sur ces bouchons, d'une part, la solution saline isoosmotique utilisée conformément à l'invention et, d'autre part, les produits connus suivants, à savoir :
- celui commercialisé sous la marque AUDISPRAY, par la Société DIEPHA, s'agissant d'une préparation à base d'eau de mer purifiée et stérilisée,
- celui commercialisé sous la marque C-FLUID par le Laboratoire LCO, s'agissant d'une solution aqueuse naturelle comprenant des sels de calcium, de sodium et de magnésium sous forme ionisée,
- celui commercialisé en tant que médicament sous la marque CERUMENOL par les Laboratoires Jean-Paul MARTIN, s'agissant d'une solution de polysorbate 80 à 0,5%,
- celui commercialisé en tant que médicament sous la marque CÉRULYSE par le Laboratoire CHAUVIN SA, s'agissant d'un produit qui contient du xylène en tant que principe actif,
- celui commercialisé sous la forme de préparation d'hygiène sous la marque A CÉRUMEN par les Laboratoires GILBERT, s'agissant d'un produit à base de PEG-120 méthylglucose dioléate de bétaïne et d'alkylamidopolypeptide; sel de TEA,
- celui commercialisé sous la forme d'un médicament vendu sous la marque OTOLYSINE par le Laboratoire CHAUVIN, s'agissant d'un médicament à base de caprylate de triéthanolamine et d'acide caprylique.

Deux séries d'expériences ont été effectuées; la première portait sur des cérumens de jeunes adultes et la deuxième sur des cérumens de personnes âgées.

Dans la première série d'expériences, on a dilué dans des tubes à hémolyse et avec incubation à 37°C, des quantités de respectivement 0,1 g de bouchon de cérumen dans respectivement 5 ml, d'une part, de la solution saline isoosmotique utilisée conformément à l'invention (solution A) et, d'autre part, de chacun des produits identifiés ci-dessous, à savoir :

| | |
|---|---|
| AUDISPRAY | Solution B |
| C-FLUID | Solution C |
| CÉRUMENOL | Solution D |
| CÉRULYSE | Solution E |
| A CÉRUMEN | Solution F |
| OTOLYSINE | Solution G |

Dans la deuxième série d'expériences, on a procédé de la même façon avec des quantités de 0,1 g de cérumen dans 5 ml de la solution isoosmotique utilisée conformément d'une part à l'invention (Solution A') et d'autre part dans 5 ml des produits identifiés ci-dessous :

| | |
|---|---|
| AUDISPRAY | Solution B' |
| CÉRUMENOL | Solution D' |
| CÉRULYSE | Solution E' |
| A CÉRUMEN | Solution F' |
| OTOLYSINE | Solution G' |

Dans les deux séries d'expériences, on a analysé l'effet lytique des différentes solutions (prise de photos à 0 min, 1 min, 3 min, 5 min, 15 min; 30 min et 45 min), étant entendu qu'avant chaque prise de photo les tubes sont retournés une fois pour rendre visible la dissociation du bouchon.

Afin de vérifier l'effet lytique et de trouver les conditions optimales de prises de vue, ces expériences ont été répétées trois fois avec des quantités moindres de la matière constituée de bouchons de jeunes patients en conservant les-concentrations ci-dessus ; en d'autres termes, on a utilisé des quantités de 0,02g pour 1 ml de produit céruménolytique.

L'examen des photos correspondantes montre la supériorité de la solution saline isoosmotique utilisée selon l'invention.

L'effet céruménolytique de la solution saline, isoosmotique utilisée, conformément à l'invention et des produits céruménolytiques de l'art antérieur identifiés ci-dessus a également été analysé par densitométrie (il s'agit d'analyses effectuées à l'aide du densitomètre BIORAD, des images numérisées correspondant aux différentes solutions A à G et A' à G', prises en photo), ceci pour évaluer l'effet de dissolution des bouchons de cérumen par l'examen du trouble qui apparaît dans les tubes. Plus la valeur de la densitométrie mesurée est grande, plus le trouble est important et plus l'effet céruménolytique est grand.

Les résultats des mesures densitométriques sont réunis dans les tableaux B et C ci-après.

Le tableau B concerne la première série d'essais.

**TABLEAU B**

| Produit testé | Résultats des mesures densitométriques | | | | | |
|---|---|---|---|---|---|---|
| | T=O | T=3 | T=5 | T=15 | T=30 | T=45 |
| Solution A | 225 | 226 | 254 | 405 | 557 | 652 |
| Solution B | 232 | 235 | 267 | 310 | 464 | 510 |
| Solution C | 212 | 215 | 228 | 265 | 424 | 448 |
| Solution D | 207 | 225 | 260 | 397 | 524 | 590 |
| Solution E | 219 | 237 | 242 | 285 | 344 | 356 |
| Solution F | 197 | 210 | 241 | 320 | 383 | 398 |
| Solution G | 211 | 225 | 239 | 314 | 377 | 402 |

Dans ce tableau, on a indiqué pour chaque solution la valeur mesurée de la densitométrie au temps 0 (T=0), puis après 3 min (T=3), 5 min (T=5), 15 min (T=15), 30 min (T=30) et 45 min (t=45).

Le tableau C qui est constitué de la même façon que le tableau B réunit les résultats de la deuxième série d'expérience.

**TABLEAU C**

| Produit testé | Résultats des mesures densitométriques | | | | | |
|---|---|---|---|---|---|---|
| | T=0 | T=3 | T=5 | T=15 | T=30 | T=45 |
| Solution A' | 218 | 226 | 254 | 297 | 352 | 378 |
| Solution B' | 183 | 183 | 223 | 233 | 235 | 244 |
| Solution D' | 189 | 189 | 190 | 224 | 290 | 350 |
| Solution E' | 185 | 185 | 185 | 190 | 205 | 211 |
| Solution F' | 170 | 170 | 193 | 199 | 207 | 211 |
| Solution G' | 183 | 183 | 183 | 205 | 210 | 244 |

Les résultats réunis dans les tableaux B et C font clairement apparaître la supériorité de la solution saline isoosmotique selon l'invention.

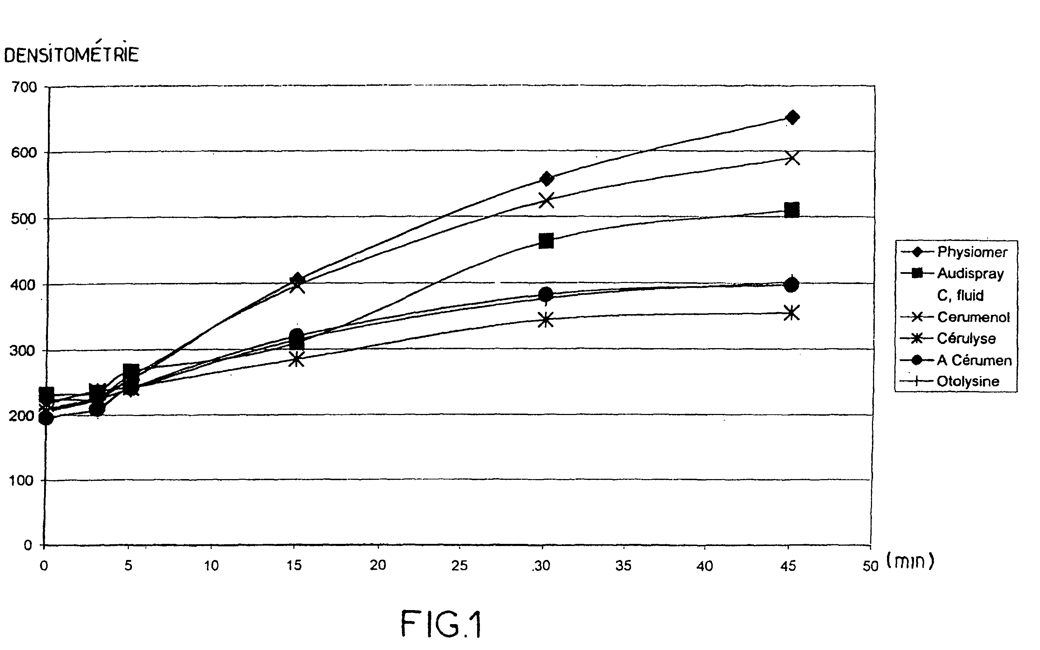

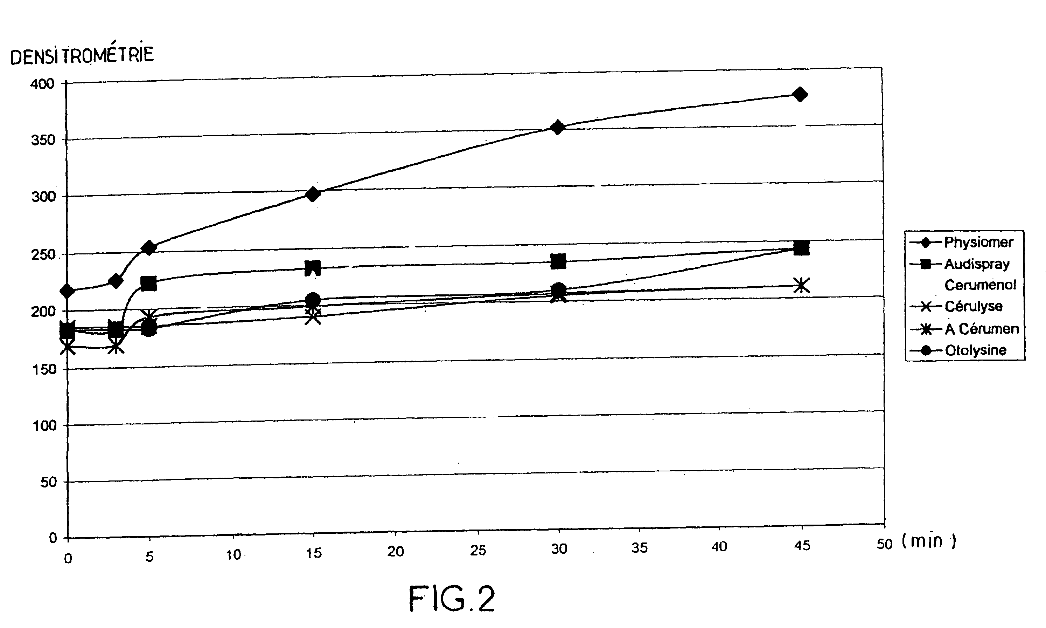

Pour la commodité de la comparaison des résultats réunis dans les tableaux B et C, on a transposé les résultats de ces tableaux respectivement sur les graphiques des figures 1 et 2 qui montrent la variation de la densitométrie en fonction du temps exprimé en minutes.

Les nouveaux médicaments céruménolytiques conformes à l'invention peuvent être appliqués par pulvérisation ou par instillation sous forme de gouttes dans le conduit auditif externe de l'oreille.

En présence de bouchons de cérumen, on peut avantageusement les appliquer à raison de deux pulvérisations ou instillations quotidiennes pendant une période de 3 à 4 jours.

A titre d'hygiène régulière, on peut les appliquer avantageusement deux à trois fois par semaine, et plus dans le cas des porteurs de prothèses auditives, afin de favoriser le bon fonctionnement de l'appareil.

## Revendications

1. Utilisation de solutions salines isoosmotiques, notamment obtenues à partir de l'eau de mer, pour l'obtention d'un médicament destiné à un traitement céruménolytique.

2. Utilisation selon la revendication 1 d'une solution saline isoosmotique **caractérisée :**
- **par** un pH de 7,8 à 8,3
- **par** une densité de 1,008 à 1,01
- **par** une teneur en matières sèches de 1 à 2% en poids
- **par** une osmolarité de 305 à 315 mOs/kg et
- **par** une constitution chimique résultant, pour ce qui est de ses principaux éléments, du tableau A ci-après :
**TABLEAU A**
| | |
|---|---|
| Solution (Na) | de 2000 à 2600 mg/l |
| Polassium (K) | de 40 à 80 mg/l |
| Chlorures (Cl) | de 5800 à 600 mg/l |
| Calcium (Ca) | de 300 à 400 mg/l |
| Magnésium (Mg) | de 1200 à 1500 mg/l |

## Claims

1. Use of isoosmotic saline solutions, in particular obtained from sea water, for obtaining a medicine which is intended for a cerumenolytic treatment.

2. Use according to Claim 1 of an isoosmotic saline solution, **characterized:**
- **by** a pH of from 7.8 to 8.3
- **by** a density of from 1.008 to 1.01
- **by** a dry matter content of from 1 to 2% by weight,
- **by** an osmolarity of from 305 to 315 mOs/kg, and
- **by** a chemical composition which is evident, as far as its main elements are concerned, from table A below:
**TABLE A**
| | |
|---|---|
| Solution (Na) | from 2 000 to 2 600 mg/l |
| Potassium (K) | from 40 to 80 mg/l |
| Chlorides (Cl) | from 5 800 to 600 mg/l |
| Calcium (Ca) | from 300 to 400 mg/l |
| Magnesium (Mg) | from 1 200 to 1 500 mg /l |

## Patentansprüche

1. Verwendung von isoosmotischen Salzlösungen, die insbesondere aus Meerwasser gewonnen werden, zur Erlangung eines Medikaments, das für eine Ohrenschmalzbehandlung bestimmt ist.

2. Verwendung nach Anspruch 1 einer isoosmotischen Salzlösung, **gekennzeichnet:**
- **durch** einen pH-Wert von 7,8 bis 8,3,
- **durch** eine Dichte von 1,008 bis 1,01,
- **durch** einen Trockenmassenanteil von 1 bis 2% im Gewicht,
- **durch** eine Osmolarität von 305 bis 315 mOsmol/kg, und
- **durch** eine chemische Zusammensetzung, die sich, was ihre hauptsächlichen Elemente angeht, aus der nachfolgenden Tabelle A ergibt:
**TABELLE A**
| | |
|---|---|
| Natrium (Na) | von 2000 bis 2600 mg/l |
| Kalium (K) | von 40 bis 80 mg/l |
| Chloride (Cl) | von 5800 bis 600 mg/l |
| Calzium (Ca) | von 300 bis 400 mg/l |
| Magnesium (Mg) | von 1200 bis 1500 mg/l |
